# EUROPEAN PATENT SPECIFICATION

(11) **EP 2 234 556 B1**
(45) Date of publication and mention of the grant of the patent: **11.11.2015**
(21) Application number: 08705586.9
(22) Date of filing: 11.01.2008
(51) Int. Cl.: A61B 18/04, A61B 17/32

(54) **ULTRASONIC WOUND DEBRIDER PROBE**
ULTRASCHALLSONDE FÜR WUND-DEBRIDER
SONDE À ULTRASONS DE DÉBRIDEMENT DE PLAIES

(43) Date of publication of application: 06.10.2010
(73) Proprietor: Misonix Incorporated, Farmingdale, NY 11735 (US)
(72) Inventor: BUSH, Christopher, Commack, NY 11725 (US); NOVAK, Theodore, A.D., Northport NY 11768 (US)
(74) Representative: Dall'Olio, Giancarlo
(86) International application number: PCT/US2008/000460
(87) International publication number: WO 2009/091352

(56) References cited:
- EP-A1- 0 897 696
- EP-A1- 1 138 264
- WO-A1-2006/092576
- WO-A2-02/060525
- AU-A1- 2003 241 752
- US-A1- 2004 030 254
- US-A1- 2006 241 470
- US-A1- 2008 004 649
- US-B2- 7 025 735

## Description

### BACKGROUND OF THE INVENTION

This invention relates to ultrasonic surgical instruments More particularly, this invention relates to high-efficiency medical treatment probes for ultrasonic surgical aspirators. These probes increase the ability to fragment and emulsify hard and soft tissue in a clinical environment while reducing unwanted heat and collateral tissue damage.

Over the past 30 years, several ultrasonic tools have been invented which can be used to ablate or cut tissue in surgery. Such devices are disclosed by Wuchinich et al. in U.S. Patent No. 4,223,676 and Idemoto et al in U.S. Patent No. 5,188,102.

In practice, these surgical devices include a blunt tip hollow probe that vibrates at frequencies between 20 kiloHertz and 100 kiloHertz, with amplitudes up to 300 micrometers or more. Such devices ablate tissue by producing cavitation bubbles which implode and disrupt cells, by generating tissue compression and relaxation stresses (sometimes called the jackhammer effect), or by giving rise to other forces such as mechanical shearing and micro streaming of bubbles in the tissue matrix. The effect is that the tissue becomes fragmented and separated. It then becomes emulsified with the irrigant solution. The resulting emulsion is then aspirated from the site. Bulk excision of tissue is possible by applying the energy around and under an unwanted tissue mass to separate it from the surrounding structure. The surgeon can then lift the tissue out using common tools such as forceps.

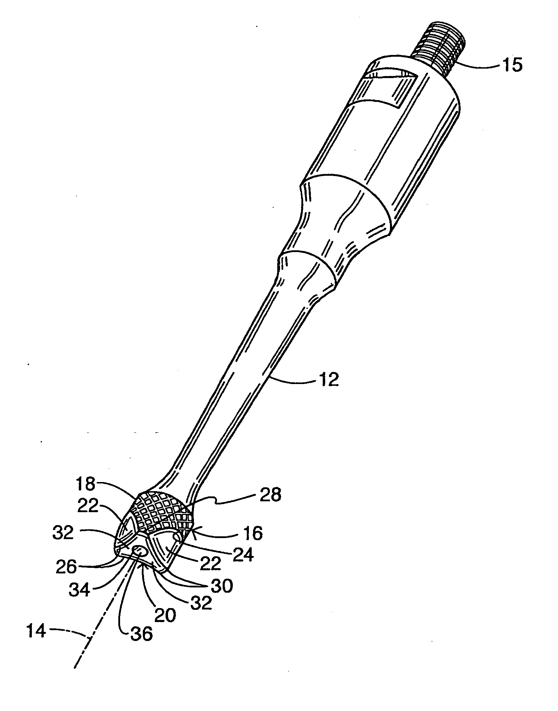

The probe or tube is excited by a transducer of either the piezoelectric or magnetostrictive type that transforms an alternating electrical signal within the frequencies indicated into a longitudinal or transverse vibration. When the probe is attached to the transducer, the two become a single element with series and parallel resonances. The designer will try to tailor the mechanical and electrical characteristics of these elements to provide the proper frequency of operation. Most of the time, the elements will have a long axis that is straight and has the tip truncated in a plane perpendicular to the long axis, as shown in Fig 1. This is done for simplicity and economic considerations. In almost all applications, whether medical or industrial, such an embodiment is practical and useful. However, in applications such as the debridement of burns, wounds, diabetic ulcers or ulcers induced by radiation treatments, the blunt straight probe has been shown to be less effective in removing the hard eschar buildup that occurs when the wound is healing. This eschar buildup must be removed so that the healthy tissue is exposed and allowed to close the wound to provide complete healing with minimal scar tissue formation. Also, the small diameter tip, since it is cannulated, has a small annular area with limits energy transmission into the wound. This extends the length of the procedure and causes operator fatigue and patient discomfort.

Therefore, it is desired to provide a probe that can be mated to an ultrasonic surgical aspirator which increases the efficiency of emulsification, does not heat up the operative site and lowers the time of operation.

WO 2006/092576 discloses a ultrasonic surgical tool having an elongate waveguide operatively connected at a proximal end to a source of ultrasonic vibrations. At a distal end, an operative element comprises a radially-extending ridge defined between a substantially parallel pair of grooves extending longitudinally of the waveguide. When a distal end surface is perpendicular to the axis of the waveguide the distal end comprises two concave shaping surfaces and two planar shaping surfaces.

The preamble of claim 1 is based on this document which is considered the closest prior art. AU2003241752 discloses a curved ultrasonic blade including a concave top surface, a convex bottom surface and a central ridge running along the concave top surface is described. The curved blade the blade has a cross section which is substantially trapezoidal. The blade edges may be sharp or blunt and the convex bottom surface has a width of at least two times the width of the central fridge.

According to AU2003241752 a balanced ultrasonic surgical instrument must include an ultrasonic transmission rod which is connected to the curved blade by a balance portion which includes first and second balance asymmetries designed to compensate for the imbalances induced by asymmetry of the curved blade. EP1138264 discloses an ultrasonic surgical blade including a top surface, a bottom surface and a cutting-edge.

The cutting-edge is defined by a cutting-surface intermediate the top surface and the bottom surface, and the top surface has a width greater than the width of the bottom surface.

### SUMMARY OF THE INVENTION

The present invention aims to provide an improved ultrasonic surgical instrument for use in debridement of wounds in the form of a probe that may be used in conjunction with ultrasonic surgical aspirators to debride wounds. The improved ultrasonic surgical instrument has a form that enhances surgical efficiency and reduces the time required to complete at least some kinds of debridement procedures. The ultrasonic surgical instrument preferably has irrigation and/or suction capability. The ultrasonic surgical instrument may be used in debriding deep wounds such as cuts and puncture wounds.

An ultrasonic probe in accordance with the present invention comprises a shaft having a longitudinal axis and a head disposed at a distal end of the shaft. The head has a cylindrical lateral surface and a substantially planar end face oriented substantially perpendicular relative to the axis. The head has at least three shaping surfaces at a distal end of the cylindrical surface, each of the shaping surfaces extending at a respective acute angle to the axis. Each of the shaping surfaces is intersecting or contiguous with both the cylindrical surface and the end face.

Pursuant to further features of the present invention, the shaping surfaces are angularly or circumferentially spaced from each other about the axis, while the end face has a substantially polygonal shape.

Pursuant to additional feature of the present invention, the end face has a plurality of outer edges each contiguous with two adjacent edges at sharp points, while the shaping surfaces are concave, so that the outer edges of the end face include a plurality of concave edges each along a respective one of the shaping surfaces and further include a plurality of convex edges along the cylindrical surface and between the shaping surfaces.

Pursuant to other features of the present invention, the cylindrical surface is knurled, the end face has at least three arms extending radially outwardly from a hub area, the end face has at least three outer edges each contiguous with two adjacent ones of the outer edges at sharp points, and the shaping surfaces are concave so that the end face has a scalloped shape.

In a specific embodiment of the invention, the shaping surfaces are exactly three in number, while the end face has six outer edges each contiguous with two adjacent ones of the outer edges at sharp points.

According to the invention, the shaping surfaces are concave. In that event, the outer edges of the probe end face includes three concave edges and three convex edges, the concave edges alternating with the convex edges about the periphery of the end face. Each concave edge is disposed along a respective one of the shaping surfaces, while each convex edge is disposed along the cylindrical surface and between the shaping surfaces.

An ultrasonic probe more generally comprises, in accordance with the present invention, a shaft having a longitudinal axis and a head disposed at a distal end of the shaft. The head has (a) at least one lateral surface that is oriented substantially parallel to the axis, (b) a substantially planar end face oriented substantially perpendicularly relative to the longitudinal axis, and (c) at least three shaping surfaces each extending at a respective acute angle to the axis and each intersecting or contiguous with the lateral surface and the end face. The end face has a substantially polygonal shape.

The shaping surfaces may be angularly or circumferentially equispaced from each other about the axis of the probe, while the end face may have six outer edges each contiguous with two adjacent ones of the outer edges at sharp points.

In additional aspects of the present invention, the lateral surface is knurled, the end face has at least three arms extending radially outwardly from a hub area, the end face has at least three outer edges each contiguous with two adjacent ones of the outer edges at sharp points, and the shaping surfaces are concave so that the end face has a scalloped shape.

Where the shaping surfaces are concave, the outer edges include multiple concave edges each along a respective one of the shaping surfaces and further including multiple edges between the shaping surfaces.

### BRIEF DESCRIPTION OF THE DRAWING

The sole FIGURE of the drawing is a perspective view of an ultrasonic wound debrider probe in accordance with the present invention.

### DETAILED DESCRIPTION

An ultrasonic probe as shown in the drawing includes a shaft 12 having a longitudinal axis 14. At a proximal end of the shaft is provided a screw-type connector 15 for coupling the probe to an ultrasonic transducer assembly in a handpiece (neither shown). A cylindrical or barrel-shaped head 16 is disposed at a distal end of shaft 12. Head has a knurled cylindrical lateral surface 18 and an end face 20 oriented transversely or perpendicularly to axis 14. Head 16 is formed with three concave shaping surfaces 22 at a distal end of lateral surface 18. Each of the shaping surfaces extends at a respective acute angle to axis 14. Accordingly, each shaping surface 22 is inclined relative to lateral surface 18 and end face 20. Each shaping surface 22 intersects or is contiguous with cylindrical surface 18 along an arcuate groin line 24. Each shaping surface 22 intersects or is contiguous with end face 20 along a concave edge 26.

Shaping surfaces 22 are angularly or circumferentially spaced from each other about axis 14, so that end face 20 is bounded not only by concave edges 26 but also by convex edges 28 in the form of circular sections. Concave edges 26 and convex edges 28 are collectively outer edges of end face 20. Each concave outer edge 26 is contiguous with two adjacent convex outer edges 28 at a pair of sharp points 30. Similarly, each convex outer edge 28 is contiguous with two adjacent concave outer edges 26 at sharp points 30.

End face 20 has a generally polygonal shape. In other words, rather than being characterized by a smoothly continuous outer edge such as a circle, an ellipse, or oval, the periphery of end face 20 is characterized by a plurality of sharp points where smoothly continuous edges 26 and 28 meet or join one another.

Accordingly, end face 20 has a plurality of outer edges 26 and 28 each contiguous with two adjacent edges 28 and 26 at sharp points. Shaping surfaces 22 are concave, so that the outer edges of end face 20 include a plurality of concave edges 26 each along a respective one of the shaping surfaces 22 and further include a plurality of convex edges 28 along the lateral surface 18 and between adjacent shaping surfaces 22.

As indicated above, cylindrical lateral surface 18 is knurled, that is, formed with a dense array of projections and recesses.

End face 20 has a scalloped shape with three arms 32 extending radially outwardly from a hub area 34. Hub area 34 is pierced by a distal end of a longitudinal channel or bore 36 extending through shaft 12 for purposes of delivering a liquid coolant to a surgical site.

In the illustrated embodiment, the shaping surfaces 22 are exactly three in number, while end face 20 has six outer edges 26 and 28 each contiguous with two adjacent ones of the outer edges at sharp points 30. The shaping surfaces shape the end face 20 and provide it with multiple sharp edges 26 and 28 and points 30 that aid in tissue fragmentation and would debridement.

It is possible to provide four or more shaping surfaces 22, but three is considered an optimal number.

## Claims

1. An ultrasonic probe comprising: a shaft (12) having a longitudinal axis (14); and a head (16) disposed at a distal end of said shaft (12), said head (16) having a lateral cylindrical surface (18), a substantially planar end face (20) , and a plurality of concave shaping surfaces (22) at a distal end of said cylindrical surface (18), each of said shaping surfaces (22) intersecting or contiguous with said cylindrical surface (18), **characterized in that** said head has at least three of said concave shaping surfaces (22), and **in that** said end face (20) is oriented substantially perpendicularly to said longitudinal axis (14).

2. The probe defined in claim 1, further **characterized in that** said shaping surfaces (22) are angularly or circumferentially equispaced from each other about said axis (14).

3. The probe defined in any of the preceding claims wherein said end face (20) has a generally polygonal shape, further **characterized in that** said shaping surfaces (22) are exactly three in number, said end face (20) has six outer edges (26, 28) each contiguous with two adjacent ones of said outer edges (26, 28) at sharp points (30), said outer edges (26, 28) include three concave edges (26) each along a respective one of said shaping surfaces (22), and said outer edges (26, 28) further include three convex edges (28) along said cylindrical surface (18) and between said shaping surfaces (22).

4. The probe defined in any of the preceding claims, further **characterized in that** said cylindrical surface (18) is knurled.

5. The probe defined in any of the preceding claims, further **characterized in that** said end face (20) has at least three arms (32) extending radially outwardly from a hub area (34).

6. The probe defined in any of the preceding clams, further **characterized in that** said shaft (12) has a longitudinal channel or bore (36) that intersects and opens onto said end face (20).

## Patentansprüche

1. Ultraschallsonde, Folgendes beinhaltend: einen Schaft (12) mit einer Längsachse (14); und einen Kopf (16), der an einem distalen Ende des Schafts (12) angeordnet ist, wobei der Kopf (16) eine seitliche zylindrische Oberfläche (18), eine im Wesentlichen planare Endfläche (20), und mehrere konkave formgebende Flächen (22) an einem distalen Ende der zylindrischen Oberfläche (18) aufweist, wobei jede der formgebenden Flächen (22) die zylindrische Oberfläche (18) schneidet oder daran angrenzt,
**dadurch gekennzeichnet, dass** der Kopf mindestens drei der konkaven formgebenden Flächen (22) aufweist, und dadurch, dass die Endfläche (20) im Wesentlichen orthogonal zu der Längsachse (14) ausgerichtet ist.

2. Sonde nach Anspruch 1, ferner **dadurch gekennzeichnet, dass** die formgebenden Flächen (22) in winkel- oder umfangsmäßig gleichen Abständen voneinander um die Achse (14) angeordnet sind.

3. Sonde nach einem der vorhergehenden Ansprüche, worin die Endfläche (20) eine im Allgemeinen polygonale Form aufweist, ferner **dadurch gekennzeichnet, dass** die Anzahl der formgebenden Flächen (22) genau drei beträgt, wobei die Endfläche (20) sechs Außenkanten (26, 28) aufweist, die jeweils an spitzen Punkten (30) an zwei benachbarte der Außenkanten (26, 28) angrenzen, wobei die Außenkanten (26, 28) drei konkave Kanten (26) beinhalten, die sich jeweils entlang einer der formgebenden Flächen (22) erstrecken, und die Außenkanten (26, 28) ferner drei konvexe Kanten (28) aufweisen, die sich entlang der zylindrischen Oberfläche (18) und zwischen den formgebenden Flächen (22) erstrecken.

4. Sonde nach einem der vorhergehenden Ansprüche, ferner **dadurch gekennzeichnet, dass** die zylindrische Oberfläche (18) gerändelt ist.

5. Sonde nach einem der vorhergehenden Ansprüche, ferner **dadurch gekennzeichnet, dass** die Endfläche (20) zumindest drei Arme (32) aufweist, die sich von einem Nabenbereich (34) aus radial nach außen erstrecken.

6. Sonde nach einem der vorhergehenden Ansprüche, ferner **dadurch gekennzeichnet, dass** der Schaft (12) eine(n) längsgerichtete(n) Kanal oder Bohrung (36) aufweist, welche(r) die Endfläche (20) schneidet und sich an dieser öffnet.

## Revendications

1. Une sonde à ultrasons comprenant: un arbre (12) ayant un axe longitudinal (14) ; et une tête (16) disposée au niveau d'une extrémité distale dudit arbre (12), ladite tête (16) ayant une surface cylindrique latérale (18), une face d'extrémité (20) essentiellement plate, et une pluralité de surfaces concaves de mise en forme (22) au niveau d'une extrémité distale de ladite surface cylindrique (18), chacune desdites surfaces de mise en forme (22) coupant ou étant contiguë à ladite surface cylindrique (18), **caractérisée en ce que** ladite tête a au moins trois desdites surfaces concaves de mise en forme (22), et **en ce que** ladite face d'extrémité (20) est orientée essentiellement perpendiculairement audit axe longitudinal (14).

2. La sonde selon la revendication 1, **caractérisée en outre en ce que** lesdites surfaces de mise en forme (22) sont angulairement ou circonférentiellement équidistantes les unes des autres autour dudit axe (14).

3. La sonde selon l'une quelconque des revendications précédentes, dans laquelle ladite face d'extrémité (20) a une forme généralement polygonale, **caractérisée en outre en ce que** lesdites surfaces de mise en forme (22) sont exactement au nombre de trois, ladite face d'extrémité (20) a six arêtes extérieures (26, 28) contiguës, chacune, à deux arêtes adjacentes desdites arêtes extérieures (26, 28) au niveau de pointes aiguës (30), lesdites arêtes extérieures (26, 28) comprennent trois arêtes concaves (26) chacune le long d'une surface respective desdites surfaces de mise en forme (22), et lesdites arêtes extérieures (26, 28) comprennent en outre trois arêtes convexes (28) le long de ladite surface cylindrique (18) et entre lesdites surfaces de mise en forme (22).

4. La sonde selon l'une quelconque des revendications précédentes, **caractérisée en outre en ce que** ladite surface cylindrique (18) est moletée.

5. La sonde selon l'une quelconque des revendications précédentes, **caractérisée en outre en ce que** ladite face d'extrémité (20) a au moins trois bras (32) s'étendant radialement vers l'extérieur depuis une zone moyeu (34).

6. La sonde selon l'une quelconque des revendications précédentes, **caractérisée en outre en ce que** ledit arbre (12) a un canal ou alésage longitudinal (36) qui coupe et débouche sur ladite face d'extrémité (20).
